**Europäisches Patentamt**

**European Patent Office**

**Office européen des brevets**

(19)

(11) Veröffentlichungsnummer : **0 133 911**
**B1**

(12) **EUROPÄISCHE PATENTSCHRIFT**

(45) Veröffentlichungstag der Patentschrift :
27.05.87

(21) Anmeldenummer : **84107637.5**

(22) Anmeldetag : **02.07.84**

(51) Int. Cl.⁴ : **C 07 C 21/14**, C 07 C 17/02

(54) Verfahren zur Herstellung von trans-1,1,2,3,4,4-Hexabrom-2-buten (HBB) durch Bromierung von Diacetylen.

(30) Priorität : **25.08.83 DE 3330608**

(43) Veröffentlichungstag der Anmeldung :
**13.03.85 Patentblatt 85/11**

(45) Bekanntmachung des Hinweises auf die Patenterteilung : **27.05.87 Patentblatt 87/22**

(84) Benannte Vertragsstaaten :
**AT BE CH DE FR GB IT LI NL SE**

(56) Entgegenhaltungen :
**DE-A- 2 157 538**
**US-A- 4 107 230**
**CHEMISCHES ZENTRALBLATT, Band 96, Nr. 4, 1925,
Seite 390, Berlin; LESPIEAU et al.: "Über Diacetylenhexabromid"**

(73) Patentinhaber : **HÜLS AKTIENGESELLSCHAFT
- RSP Patente / PB 15 - Postfach 13 20
D-4370 Marl 1 (DE)**

(72) Erfinder : **Zillmann, Reinhold
Ahornstrasse 23
D-4421 Reken (DE)**

EP 0 133 911 B1

## Beschreibung

Die Erfindung betrifft ein Verfahren zur gefahrlosen Herstellung von trans-1.1.2.3.4.4-Hexabrom-2-buten (HBB) durch Bromierung von Diacetylen.

HBB besitzt ausgezeichnete flammhemmende Eigenschaften und ist daher eine für die Herstellung schwer entflammbarer Kunststoffe gesuchte Verbindung. Bisher ist jedoch kein technisch angewandtes Verfahren bekannt, diese Verbindung auf gefahrlose Art durch Bromierung von Diacetylen herzustellen.

Diacetylen wird aufgrund seiner Neigung zum explosionsartigen Zerfall nahezu ausschließlich zur Herstellung von Laborpräparaten in entsprechend kleinen Mengen benutzt. So findet man in der Literatur die Empfehlung, bei Umsetzungen mit Diacetylen aus Sicherheitsgründen höchstens 10 g Diacetylen einzusetzen (Shostakovskii und Bogdanova « The Chemistry of Diacetylenes » 1974, Keter Publishing House Jerusalem Ltd.).

In der DE-AS-21 57 538 (= GB-PS-1 360 050) « Verfahren zur Herstellung polychlorierter oder polybromierter aliphatischer Kohlenwasserstoffe » wird die Halogenierung von Mischungen gasförmiger Acetylenverbindungen beschrieben. Dieses Verfahren ist jedoch in weiten Bereichen, die innerhalb der Explosionsgrenzen liegen, undurchführbar. Nach den Angaben dieser Schrift erhält man neben vielen anderen Halogenkohlenwasserstoffen auch ein Hexabrombuten-Isomeres.

Ein großer Nachteil dieses Verfahrens besteht in dem hohen Anfall flüssiger Halogenierungsprodukte, welche toxikologisch äußerst bedenklich sind. Zudem ist mit dem Anfall dieser wertlosen Verbindungen ein entsprechender Halogenverlust verbunden. Äußerst nachteilig und bedenklich bei diesem Verfahren ist auch die Art, in der die zu halogenierende Mischung der Acetylene gewonnen wird. Man leitet einen Gasstrom, der u. a. Diacetylen, Vinylacetylen, Methylacetylen, Acetylen und Butadien enthält, so lange über Aktivkohle, bis die Kohle gesättigt ist. Hierbei wird ein Gemisch an der A-Kohle adsorbiert, welches nach den Angaben des Beispiels 1 wie folgt zusammengesetzt ist :

| | |
|---|---|
| Diacetylen : | 58,5 Mol-% |
| Vinylacetylen : | 34,0 Mol-% |
| Methylacetylen und Allen : | 3,3 Mol-% |
| Acetylen : | 2,8 Mol-% |
| Butadien : | 1,4 Mol-% |

Solch hohe Konzentrationen der höheren Acetylene lassen sich nicht sicher technisch handhaben. Auch fördert die bei der Adsorption an der A-Kohle freiwerdende Adsorptionsenthalpie die Polymerisation dieser Acetylene. Polymerisate des Diacetylens explodieren in der Wärme oder bei längerem Lagern (Shostakovskii und Bogdanova « The Chemistry of Diacetylenes » 1974, Keter Publishing House Jerusalem Ltd.).

Hieraus ergab sich die Aufgabe, ein sicher durchführbares Verfahren zur Herstellung von trans-1.1.2.3.4.4-Hexabrom-2-buten durch Bromierung von Diacetylen bei geringstmöglichem Anfall anderer Bromkohlenwasserstoffe sowie geringstmögliohem Bromverlust auszuarbeiten.

Diese Aufgabe wird erfindungsgemäß entsprechend den Angaben der Patentansprüche gelöst, wobei man überraschenderweise das gewünschte trans-1.1.2.3.4.4-Hexabrom-2-buten in hoher Ausbeute und Reinheit erhält. In der Absorptionskolonne werden die das Diacetylen sowie weitere Acetylene wie Vinylacetylen und Methylacetylen enthaltenden Restkohlenwasserstoffe bei Temperaturen von — 20 °C bis + 20 °C, vorzugsweise — 15 °C bis 0 °C, insbesondere — 10 °C bis — 5 °C, sowie Drücken von 1 bar bis 2 bar, vorzugsweise bei 1 bis 1,5 bar, mit einem Hydroxylgruppen-haltigen Lösungsmittel, beispielsweise Methanol, gewaschen. Die ablaufende Lösung enthält an gelösten Gasen im wesentlichen Diacetylen, Vinylacetylen und Methylacetylen. Im allgemeinen setzt man 0,000 5 bis 0,003 Vol.Teile des Hydroxyl-gruppen-haltigen Lösungsmittels/Vol.Teil der Restkohlenwasserstoffe ein, vorzugsweise 0,001 bis 0,002 Vol.Teil des Lösungsmittels/Vol.Teil der Restkohlenwasserstoffe. Als Hydroxylgruppen-haltige Lösungsmittel eignen sich beispielsweise Methanol und/oder Ethanol.

Diese Lösung wird mit einem inerten Gas, wie z. B. Stickstoff oder Methan in einer ersten Desorptionsstufe bei Temperaturen von 0 °C bis 30 °C, vorzugsweise 10 bis 25 °C, insbesondere bei 20 °C, und Drücken von 1 bis 2 bar, vorzugsweise 1,1 bis 1,5 bar, insbesondere 1,2 bar, desorbiert, wobei die Gasphase eine relativ hohe Konzentration der mitgelösten Acetylene, insbesondere Vinylacetylen, gegenüber dem Diacetylen enthält.

Man setzt im allgemeinen 30 bis 100 Vol.Teile Inertgas/Vol.Teil Lösung ein, bevorzugt 50 bis 70 Vol.Teile Inertgas/Vol.Teil Lösung. Als Inertgase eignen sich beispielsweise Stickstoff, Wasserstoff, die Edelgase wie Helium, Neon, Argon und gasförmige Alkane wie Methan, Ethan, Propan sowie deren Gemische.

Die ablaufende methanolische Lösung enthält relativ viel Diacetylen neben nur noch wenig Vinylacetylen und praktisch kein Methylacetylen mehr. Diese Lösung ist eine geeignetere Ausgangsmischung für die Bromierung des darin enthaltenen Diacetylens.

Die direkte Bromierung dieser Lösung führt jedoch lediglich zu flüssigen Bromierungsprodukten, wie z. B. Tetrabrombutadien, während der Anfall an Hexabrombuten nur sehr gering ist. Überraschend wurde jedoch gefunden, daß man sehr gute Ausbeuten an Hexabrombuten erhält, wenn man das Diacetylen aus der methanolischen Lösung in einer zweiten Desorptionsstufe bei Temperaturen von 0 bis 40 °C, vorzugsweise bei 10 bis 30 °C, insbesondere bei 20 bis 25 °C, und Drücken von 1 bis 2 bar, vorzugsweise bei 1,2 bis 1,6 bar, insbesondere bei 1,4 bar, mit einem inerten Gas, wie z. B. Stickstoff oder Methan, desorbiert.

Man setzt im allgemeinen 90 bis 250 Vol.Teile Inertgas/Vol.Teil Lösung ein, bevorzugt 120 bis 170 Vol.Teile Inertgas/Vol.Teil Lösung. Als Inertgase eignen sich beispielsweise Stickstoff, Wasserstoff, die Edelgase wie Helium, Neon, Argon und gasförmige Alkane, wie Methan, Ethan, Propan sowie deren Gemische.

Den bei der Desorption erhaltenen Gasstrom führt man der Bromierung zu. Der Gasstrom enthält Diacetylen in einer Konzentration, die keine Gefahr mehr durch einen sonst möglichen Selbstzerfall darstelle (unter 10 %) und wesentlich weniger Vinylacetylen (ca. 10 % der Diacetylenmenge).

Die Bromierung führt man in einem Halogenkohlenwasserstoff bei Temperaturen von 20 bis 40 °C, vorzugsweise 25 bis 35 °C, insbesondere bei 30 °C, und Drücken von 1,2 bis 2 bar, vorzugsweise 1,3 bis 1,6 bar, insbesondere bei 1,4 bar, durch.

Als Halogenkohlenwasserstoffe eignen sich beispielsweise Tetrachlormethan, Tetrachlorethan und/ oder Chloroform, vorzugsweise Tetrachlormethan.

Die Bromierung kann in allgemein üblichen Reaktortypen, beispielsweise in einem Rührreaktor, stattfinden. In einer bevorzugten Ausführungsform des Verfahrens der Erfindung bromiert man in einem Blasensäulenreaktor, in dem ein Halogenkohlenwasserstoff, wie z. B. Tetrachlorethan, vorzugsweise Tetrachlormethan, mit in dem Halogenkohlenwasserstoff gelöstem Brom vorgelegt ist. Die Konzentration an gelöstem Brom beträgt im allgemeinen 0,1 bis 2 Gew.-%, vorzugsweise 0,3 bis 0,5 Gew.-%.

Führt man die Reaktion jedoch in dieser Weise durch, so stellt man nach relativ kurzer Zeit einen Rückgang der Hexabrombuten-Bildung und eine Zunahme der Bildung von flüssigen Bromierungsprodukten fest. Überraschend wurde nun gefunden, daß die Ausbeute an trans-1.1.2.3.4.4-Hexabrom-2-buten unverändert bleibt, wenn man dem Halogenkohlenwasserstoff Wasser in einer Menge von 10 bis zu 100 % seines Volumens, vorzugsweise 20 bis 40 Vol.-%, insbesondere 25 bis 35 Vol.-%, zusetzt.

Entsprechend dem Verbrauch an Brom, der gut an der Farbänderung zu beobachten ist, wird gleichzeitig mit dem Reaktionsgas Brom in einer solchen Menge zugefahren, daß der zu Beginn eingestellte Überschuß gehalten wird. Dieser Überschuß beträgt im allgemeinen 0,1 bis 2 Gew.-%, bezogen auf den Halogenkohlenwasserstoff.

Dieser Umstand bringt es mit sich, daß das Abgas geringe Mengen Brom aus dem Reaktor austrägt. Um diesen Bromverlust zu vermeiden, kann man das Abgas durch einen Nachreaktor leiten, in dem sich wie im Hauptreaktor ein chlorierter Kohlenwasserstoff als Lösungsmittel befindet. In diesen Nachreaktor wird gleichzeitig mit dem Abgas aus der Reaktion ein geringer Teilstrom des Reaktionsgases eingeleitet. Mit dem nun im Überschuß vorhandenen Diacetylen setzt man das im Abgas enthaltene Brom wiederum zu trans-1.1.2.3.4.4-Hexabrom-2-buten um, so daß das Abgas aus dem Nachreaktor keine meßbaren Brommengen mehr enthält.

Führt man die Reaktion in einem Blasensäulenreaktor durch, so füllt sich während der Fahrzeit der untere, als Auffanggefäß ausgebildete Teil des Reaktors mit dem gebildeten kristallinen trans-1.1.2.3.4.4-Hexabrom-buten und wird schließlich aus diesem zwecks Aufarbeitung abgelassen.

Zur Aufarbeitung wird das Hexabrombuten in einer geeigneten Trannvorrichtung, beispielsweise in einer Glasfilternutsche, abgesaugt, anschließend mit einem Alkohol, beispielsweise Methanol oder Ethanol, gewaschen und schließlich im Vakuum getrocknet.

Man erhält das trans-1.1.2.3.4.4-Hexabrom-2-buten mit einem Schmelzpunkt von 181 °C, der mit dem in der Literatur (Müller, Helv. 8, 831) angegebenen übereinstimmt, in hoher Reinheit bei guter Ausbeute. Das erhaltene HBB eignet sich insbesondere als flammhemmender Zusatz zu Kunststoffen.

## Beispiel 1

Man setzt ein Restgas ein, welches bei der Acetylenherstellung nach dem Lichtbogenverfahren erhalten wird und folgende Zusammensetzung hat : ①

| | |
|---|---|
| Acetylen | 0,4 Vol.-% |
| Methylacetylen | 1,8 Vol.-% |
| Diacetylen | 2,5 Vol.-% |
| Vinylacetylen | 3,1 Vol.-% |
| Allen | 0,2 Vol.-% |
| Butadien | 0,2 Vol.-% |
| Cyclopentadien | 0,9 Vol.-% |
| Ethylen | 1,7 Vol.-% |
| $C_3$-$C_4$-Olefine | 8,0 Vol.-% |
| $C_1$-$C_4$-Alkane | 72,5 Vol.-% |

| | |
|---|---|
| Aromaten | 1,3 Vol.-% |
| Wasserstoff | 5,9 Vol.-% |
| Wasser | 1,5 Vol.-% |

9,1 m³/h dieses Gases werden in dem Absorber 8 bei einer Temperatur von — 5 °C und einem Druck von 1,2 bar mit 13 l/h Methanol gewaschen.

Man erhält eine methanolische Diacetylenlösung ② mit der folgenden Zusammensetzung :

| | |
|---|---|
| Diacetylen | 5,3 Gew.-% |
| Vinylacetylen | 2,0 Gew.-% |
| Acetylen | 0,07 Gew.-% |
| Butadien | 0,2 Gew.-% |
| Cyclopentadien | 1,5 Gew.-% |
| $C_3$-$C_4$-Alkane | 1,6 Gew.-% |
| Aromaten | 11,0 Gew.-% |
| Methanol | 75,0 Gew.-% |
| Wasser | 3,3 Gew.-% |

Diese methanolische Lösung wird auf die Desorptionskolonne 9 gefahren und bei 25 °C und 1,2 bar mit 800 Nl/h Stickstoff desorbiert. Die ablaufende methanolische Lösung ③ hat die folgende Zusammensetzung :

| | |
|---|---|
| Diacetylen | 4,0 Gew.-% |
| Vinylacetylen | 0,4 Gew.-% |
| Butadien | 0,05 Gew.-% |
| Cyclopentadien | 0,3 Gew.-% |
| $C_3$- bis $C_4$-Alkane | 1,0 Gew.-% |
| Aromaten | 10,0 Gew.-% |
| Methanol | 80,0 Gew.-% |
| Wasser | 4,3 Gew.-% |

In einem zweiten Desorber 10 wird nun die Lösung ③ mit 2 500 Nl/h Stickstoff bei 20 °C und 1,5 bar desorbiert. Die ablaufende Lösung 4 wird wieder auf den Absorber 8 zurückgefahren, das Reaktionsgas ⑤ wird in den Reaktor 11 geleitet, in dem 80 l Tetrachlorkohlenstoff mit einem Bromgehalt von 0,3 Gew.-% und außerdem 20 l Wasser vorgelegt sind.

Das Reaktionsgas 5 hat folgende Zusammensetzung :

| | |
|---|---|
| Diacetylen | 4,0 Vol.-% |
| Vinylacetylen | 0,4 Vol.-% |
| Butadien | 0,08 Vol.-% |
| Cyclopentadien | 0,9 Vol.-% |
| Benzol | 1,2 Vol.-% |
| Methanol | 7,0 Vol.-% |
| Stickstoff | 86,2 Vol.-% |

Brom wird sofort aufgenommen, entsprechend dem Verbrauch wird so viel Brom in den Reaktor gepumpt, daß der Überschuß von 0,3 % gehalten wird (ca. 2,9 kg/h). Durch die Mantelkühlung des Reaktors wird die Temperatur bei etwa 30 °C gehalten. Der Druck im Reaktor beträgt 1,5 bar. Nach einer halben Stunde beginnt das trans-1.1.2.3.4.4-Hexabrom-2-buten aus der Lösung auszufallen und sammelt sich im unteren Teil des Reaktors. Nach 8 Stunden Fahrzeit wird es abgelassen und aufgearbeitet. Dazu wird es in einer Glasfilternutsche abgesaugt und anschließend mit insgesamt 5 l Methanol gewaschen. Anschließend wird es im Wasserstrahlvakuum in derselben Apparatur getrocknet.

Aus 23,5 kg Brom erhält man 20 kg trans-1.1.2.3.4.4-Hexabrom-2-buten, was einer Ausbeute von 77 % (auf Brom bezogen) entspricht.

Beispiel 2

10 m³/h eines Restgases mit der in Beispiel 1 angegebenen Zusammensetzung werden in dem Absorber 8 bei einer Temperatur von — 10 °C und einem Druck von 1,5 bar mit 20 l/h Methanol gewaschen.

Man erhält eine methanolische Diacetylenlösung 2 mit der folgenden Zusammensetzung :

| | |
|---|---|
| Diacetylen | 6,9 Gew.-% |

| | |
|---|---|
| Vinylacetylen | 2,4 Gew.-% |
| Acetylen | 0,08 Gew.-% |
| Butadien | 0,3 Gew.-% |
| Cyclopentadien | 1,7 Gew.-% |
| $C_3$-/$C_4$-Alkane | 1,6 Gew.-% |
| Aromaten | 13,0 Gew.-% |
| Methanol | 70,0 Gew.-% |
| Wasser | 4,0 Gew.-% |

Diese methanolische Lösung wird auf die Desorptionskolonne 9 gefahren und bei 25 °C und 1,4 bar mit 1 200 Nl/h Methan desorbiert. Die ablaufende methanolische Lösung 3 hat die folgende Zusammensetzung :

| | |
|---|---|
| Diacetylen | 4,8 Gew.-% |
| Vinylacetylen | 0,3 Gew.-% |
| Butadien | 0,02 Gew.-% |
| Cyclopentadien | 0,2 Gew.-% |
| $C_3$-/$C_4$-Alkane | 0,8 Gew.-% |
| Aromaten | 14,0 Gew.-% |
| Methanol | 74,0 Gew.-% |
| Wasser | 5,5 Gew.-% |

In den Desorber 10 wird nun die Lösung 3 bei 25 °C und einem Druck von 1,3 bar mit 3 500 Nl/h Methan desorbiert.

Die ablaufende Lösung 4 wird wieder auf den Absorber 8 zurückgefahren, während 3 200 Nl/h des Reaktionsgases 5 in den Reaktor 11 geleitet werden. In diesem befinden sich 60 l Chloroform mit einem Bromgehalt von 0,5 Gew.-% sowie 30 l Wasser.

Das Reaktionsgas 5 hat folgende Zusammensetzung :

| | |
|---|---|
| Diacetylen | 5,0 Vol-% |
| Vinylacetylen | 0,3 Vol-% |
| Butadien | 0,01 Vol-% |
| Cyclopentadien | 0,3 Vol-% |
| Benzol | 3,0 Vol-% |
| Methanol | 10,0 Vol-% |
| Methan | 81,4 Vol-% |

Der Druck im Reaktor beträgt 1,3 bar, die Temperatur wird bei ca. 25 °C gehalten. Die Reaktion setzt praktisch sofort ein, durch Ergänzen von ca. 3,6 kg Brom/h wird der Überschuß von etwa 0,5 Gew.-% Brom gehalten. Das Abgas 6 wird in den Nachreaktor 12 geleitet, in dem 40 l Chloroform vorgelegt sind und in den gleichzeitig 300 Nl des Reaktionsgases 5 eingeleitet werden. Das im Abgas 6 enthaltene Brom setzt sich im Nachreaktor 12 mit dem im Reaktionsgas 5 enthaltenen Diacetylen wie im Hauptreaktor 11 zu Hexabrombuten um. Das Abgas 7 aus dem Nachreaktor ist praktisch bromfrei.

Nach 8 Stunden Fahrzeit wird das gebildete Hexabrombuten sowohl aus dem Hauptreaktor 11 als auch aus dem Nachreaktor 12 abgelassen und gemeinsam aufgearbeitet. Nach dem Waschen mit ca. 5 l Methanol wird das Hexabrombuten im Vakuum getrocknet.

Aus den eingesetzten 29,5 kg Brom erhält man 26,5 kg trans-1.1.2.3.4.4-Hexabrom-2-buten vom Schmelzpunkt 182 °C, was einer Ausbeute von 81 % (auf Brom bezogen) entspricht.

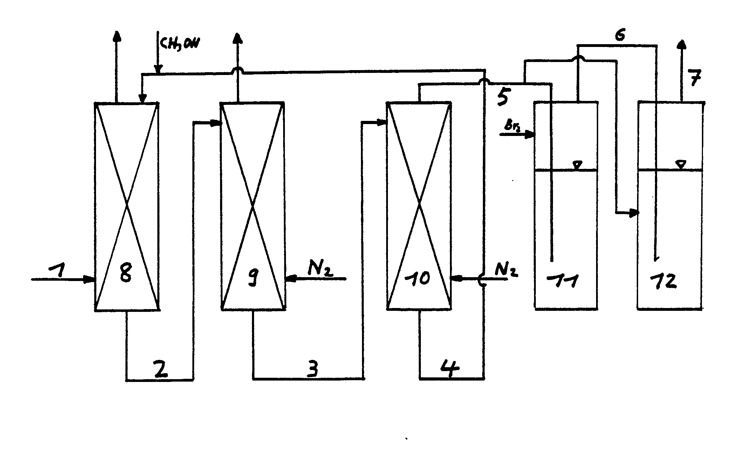

**Patentansprüche**

1. Verfahren zur Herstellung von trans-1.1.2.3.4.4-Hexabrom-2-buten durch Bromierung von Diacetylen, dadurch gekennzeichnet, daß man aus den bei der Acetylengewinnung anfallenden Restkohlenwasserstoffen das Diacetylen in einem Hydroxylgruppen-haltigen Lösungsmittel bei Temperaturen von — 20 °C bis + 20 °C und Drücken von 1 bis 2 bar löst, in einer ersten Desorptionsstufe mit einem inerten Gas bei Temperaturen von 0 bis 30 °C und Drücken von 1 bis 2 bar zunächst ein vinylacetylenreiches Gemisch abtrennt, dann in einer zweiten Desorptionsstufe mit einem inerten Gas bei Temperaturen von 0 bis 30 °C und Drücken von 1 bis 2 bar ein diacetylenreiches Gasgemisch desorbiert, dieses in einem Reaktor, in dem überschüssiges Brom in einem Halogenkohlenwasserstoff gelöst vorgelegt ist, wobei man dem Halogenkohlenwasserstoff gegebenenfalls Wasser zusetzt, bei Temperaturen von 20 bis 40 °C und Drücken von 1,2 bis 2 bar zum trans-1.1.2.3.4.4-Hexabrom-2-buten bromiert, wobei man zusätzlich mit dem Reaktionsgas gasförmiges Brom in den Reaktor gibt, und das bromhaltige Abgas gegebenenfalls in einem Nachreaktor mit überschüssigem Diacetylen in einem Halogenkohlenwasserstoff umsetzt.

5

2. Verfahren nach Anspruch 1, dadurch gekennzeichnet, daß man als Hydroxylgruppen-haltige Lösungsmittel Methanol und/oder Ethanol einsetzt.

3. Verfahren nach Anspruch 1 und 2, dadurch gekennzeichnet, daß man als Halogenkohlenwasserstoff Tetrachlorkohlenstoff und/oder Chloroform einsetzt.

4. Verfahren nach den Ansprüchen 1 bis 3, dadurch gekennzeichnet, daß man dem Halogenkohlenwasserstoff Wasser in einer Menge von 10 bis 100 Vol.-%, vorzugsweise 20 bis 40 Vol.-%, zusetzt.

5. Verfahren nach den Ansprüchen 1 bis 4, dadurch gekennzeichnet, daß man das Diacetylen bei Temperaturen von 25 bis 35 °C bromiert.

6. Verfahren nach den Ansprüchen 1 bis 5, dadurch gekennzeichnet, daß man die Bromierung in einem Blasensäulenreaktor durchführt.


## Claims

1. A process for the production of trans 1,1,2,3,4,4-hexabromo-2-butene by bromination of diacetylene, characterised in that the diacetylene from the hydrocarbon residue arising in the acetylene recovery operation is dissolved at a temperature of — 20 °C to + 20 °C and a pressure of 1 to 2 bar in a solvent containing a hydroxyl group, initially a mixture rich in vinylacetylene is separated off in a first desorption stage at a temperature of 0 to 30 °C and a pressure of 1 to 2 bar with an inert gas, then a gas mixture rich in diacetylene is desorbed in a second desorption stage at a temperature of 0 to 30 °C and a pressure of 1 to 2 bar with an inert gas, this diacetylene-rich gas mixture is brominated in a reactor in which excess bromine dissolved in a halohydrocarbon has previously been placed, water optionally being added to the halohydrocarbon, at a temperature of 20 to 40 °C and a pressure of 1.2 to 2 bar to form trans 1,1,2,3,4,4- hexabromo-2-butene, gaseous bromine being additionally fed to the reactor with the reaction gas, and the bromine-containing offgas is optionally reacted in an after-reactor with excess diacetylene in a halohydrocarbon.

2. A process according to claim 1, characterised in that methanol and/or ethanol is introduced as the solvent containing a hydroxyl group.

3. A process according to claim 1 or 2, characterised in that carbon tetrachloride and/or chloroform is introduced as halohydrocarbon.

4. A process according to any of claims 1 to 3, characterised in that water is introduced into the halohydrocarbon in an amount of 10 to 100 % by volume, preferably 20 to 40 % by volume.

5. A process according to any of claims 1 to 4, characterised in that the diacetylene is brominated at a temperature of 25 to 35 °C.

6. A process according to any of claims 1 to 5, characterised in that the bromination is carried out in a bubble column reactor.


## Revendications

1. Procédé de préparation de trans-1,1,2,3,4,4-hexabromo-2-butène par bromation de diacétylène, caractérisé par le fait qu'à partir des hydrocarbures résiduels se formant lors de l'obtention de l'acétylène, on dissout le diacétylène dans un solvant renfermant des groupes hydroxyle, à des températures de — 20 °C à + 20 °C et sous des pressions de 1 à 2 bars, que dans un premier stade de désorption avec un gaz inerte, à des températures de zéro à 30 °C et sous des pressions de 1 à 2 bars, on sépare d'abord un mélange riche en vinyl-acétylène, qu'ensuite dans un second stade de désorption avec un gaz inerte, à des températures de zéro à 30 °C et sous des pressions de 1 à 2 bars, on désorbe un mélange gazeux riche en diacétylène, que l'on brome ce dernier dans un réacteur dans lequel on a placé un excès de brome en solution dans un hydrocarbure halogéné, en ajoutant éventuellement de l'eau à l'hydrocarbure halogéné, à des températures de 20 à 40 °C et sous des pressions de 1,2 à 2 bars, pour obtenir le trans-1,1,2,3,4,4-hexabromo-2-butène, tandis qu'on ajoute complémentairement dans le réacteur du brome gazeux au gaz réactionnel et que l'on fait éventuellement réagir le gaz d'échappement renfermant du brome, dans un réacteur subséquent, sur un excès de diacétylène, dans un hydrocarbure halogéné.

2. Procédé selon la revendication 1, caractérisé par le fait que l'on utilise le méthanol et/ou l'éthanol comme solvant renfermant des groupes hydroxyle.

3. Procédé selon les revendications 1 et 2, caractérisé par le fait que l'on utilise le tétrachlorure de carbone et/ou le chloroforme comme hydrocarbure halogéné.

4. Procédé selon les revendications 1 à 3, caractérisé par le fait que l'on ajoute de l'eau à l'hydrocarbure halogéné, dans une quantité de 10 à 100 % en volume, de préférence de 20 à 40 % en volume.

5. Procédé selon les revendications 1 à 4, caractérisé par le fait que l'on brome le diacétylène à des températures de 25 à 35 °C.

6. Procédé selon les revendications 1 à 5, caractérisé par le fait que l'on effectue la bromation dans un réacteur comportant des colonnes à bulles.